# EUROPÄISCHE PATENTSCHRIFT

(11) **EP 2 194 896 B1**
(45) Veröffentlichungstag und Bekanntmachung des Hinweises auf die Patenterteilung: **26.03.2014**
(21) Anmeldenummer: 08806929.9
(22) Anmeldetag: 27.08.2008
(51) Int. Cl.: A61B 17/68, A61B 17/72

(54) **Vorrichtung zur Osteosynthese von Oberarmknochenhals-Brüchen und -Verrenkungen**
Device for the osteosynthesis of fractures and luxations of the neck of the humerus
Dispositif d'ostéosynthèse à la suite de fractures et de luxations de l'humérus

(30) Priorität: 27.08.2007 RU 2007132339
(43) Veröffentlichungstag der Anmeldung: 16.06.2010
(73) Patentinhaber: Bukreev, Alexander, Adigeia 385132 (RU)
(72) Erfinder: Bukreev, Alexander, Adigeia 385132 (RU)
(74) Vertreter: Jeck, Anton
(86) Internationale Anmeldenummer: PCT/IB2008/002224
(87) Internationale Veröffentlichungsnummer: WO 2009/027797

(56) Entgegenhaltungen:
- WO-A-97/01990
- FR-A- 2 747 911
- JP-A- 2004 248 931

## Beschreibung

Die Erfindung bezieht sich auf eine Vorrichtung zur Osteosynthese von Oberarmbeinhals-Brüchen und -Verrenkungen nach dem Oberbegriff des Anspruchs 1.

Die Vorrichtung gehört zur Medizin und zwar zur Orthopädie und Traumatologie und kann bei der operativen Behandlung der verschiedenen Oberarmbeinhals-Brüche und -Verrenkungen verwendet werden (Anweisung für die innere Osteosynthese. Die Methodik, die von der Gruppe AO (Schweiz) Springer-Verlag, 2001, 650 Seiten. S. 156 - 159 empfohlen wurde).

Aus dem japanischen Patent 2004248931 A ist eine Vorrichtung zur Osteosynthese von Oberarmbein-Brüchen und Verrenkungen mit einem U-förmigen Stift aus bioniertem Metall bekannt, wobei die Schenkel des Stifts am proximalen Ende gewölbt ineinander übergehen und wobei die Schenkel des Stifts der Länge nach gewölbt sind.

Bei diesem bekannten Stift ist der Halt des Stifts im Knochen nicht ausreichend gesichert.

Das aktuelle Problem der modernen Orthopädie und Traumatologie ist eine erweiterte Erarbeitung von Stahlkonstruktionen für die operative Behandlung der Oberarmbrüche und Oberarmverrenkungen bei allen drei Arten der Brüche. Die Brüche des proximalen Endes des Oberarmbeines betragen etwa 5 % aller Brucharten. Bei der operativen Behandlung wird immer das höchst gute funktionelle Resultat geplant. Bei der Anwendung vorhandener altbekannter Stahlkonstruktionen gibt es nach der operativen Behandlung immer die Probabilität der sekundären Dislokation der Bruchstücke und andere Komplikationen. Mit der Erarbeitung neuer Stahlkonstruktionen werden die nachteile vorhandener Stahlkonstruktionen beseitigt. Die Anwendung von Metallplatten verschiedener Modifikationen ermöglicht nicht immer die Dislozierung der Bruchstücke vollständig zu beseitigen. Die Kranken stören Schmerzen und Funktionsstörungen des Oberarmbeingelenkes.

Bei der Verwendung altbekannter Stahlkonstruktionen, die durch einen umfangreichen, traumatischen nicht anatomischen Zugang begleitet werden, ist immer die Traumatisierung der Muskeln, Gefäße und Nerven möglich. Dies führt zu späteren Zweiterkrankungen, posttraumatischer, deformierter Arthrose des Armgelenkes und in einigen Fällen zu Osteonekrose, Atrophien des Schultergürtels und schwierigen Kontraktionen. Die Anwendung einer vollendeten Konstruktion vermeidet diese Komplikationen und erreicht eine frühere und präzisere Reposition der Bruchstücke mit einer sicheren und untraumatischen inneren Osteosynthese. Aufgrund der Daten der inländischen und ausländischen Literatur über die operative Handhabung der Oberarmbeinhals-Brüche und -Verrenkungen kann man bemerken, dass die offene Reposition und die innere Osteosynthese als weitverbreitete und höchst wirksame Heilar ten gelten. Unter allen vorhandenen Stahlkonstruktionen gibt es aber keine Konstruktion, die eine einfache, nicht sperrige untraumatische und sichere Fixierung ermöglicht. Bekannt ist eine Vorrichtung für die Osteosynthese der Oberarmbeinhals-Brüche und -Verenkungen mit Disloktion, die eine T-förmige Platte mit drei oder vier Löchern verwendet (M. E. Müller. Anweisung für die innere Osteosynthese. Die Methodik, die von der Gruppe AO (Schweiz) Springer-Verlag, 2001. 650 Seiten. S. 156 - 159 empfohlen wurde).

Diese Platte wird auf folgende Weise verwendet:
Der beste Zugang zum proximalen Teil des Oberarmbeines wird durch einen Schnitt längs der Rinne zwischen m. pectoralis und m. deltoideus erreicht. Der Zugang kann durch das Abtragen des Deltamuskels vom Schlüsselbein und vom Oberarmfortsatz erweitert werden.

Die stabile Fixierung wird mit Hilfe der offenen Reposition und der inneren Fixation der Platte erzielt. Die Platte muss unbedingt so liegen, dass die Funktion der langen Sehne der m. Biceps nicht verletzt wird. Für die Fixierung des Kopfstückes sind spongiöse Schrauben von 6,5 mm mit einem über die gesamte Länge reichenden Gewinde geeignet. Bei subkapitalen Brüchen liegt die Diaphyse fast immer subkutan und kann unter dem Kopf mit Hilfe eines kleinen Knochenretraktors zurück hinterlegt werden.

Nach der Operation wird die Hand in die Position der Ableitung auf die Schiene hingelegt. Die physiotherapeutische Behandlung beginnt aus dieser Lage. Man muss die langwierige Immobilisierung des Oberarmbeingelenkes vermeiden. Nachteile der extrakortikalen Osteosynthese der T-förmigen Platte sind:
1) Das Anlegen der Platte erfordert einen umfangreichen operativen Zugang und eine Freilegung des Knochens auf einer großen Strecke. Dies verstärkt die Gefahr der Infektionskomplikationen im Vergleich zur intramedullären oder außerherdförmigen Osteosynthese.
2) Die massive Platte, die auf der Knochenhaut sogar ohne ihr Abblättern aufgelegt wird, bringt Störungen der Blutversorgung. Die Platte, die mit dem Knochen auf der ganzen Oberfläche Kontakt hat, erregt seine Nekrose und generalisierte Osteoporose. Das ist eine rechtmäßige biologische Antwort des Knochens, die sich in einer beschleunigten Remodellierung seiner Systeme zeigt.
3) Die mit der Osteoporose verbundene Störung der Fertigkeit des Knochens kann zur Entstehung der Refraktur in den Stellen der Einführung der Schranken führen, wenn die Platte vor der Vollendung der Remodellierungsprozesse beseitigt wird.
4) Die hohe Probabilität der Verletzung des Nerves Axsilaris zur Zeit der Osteosynthese.

Für die nächste Analogie kann die T-Platte mit einer hakenförmigen Spitze nach K. M. Klimov gehalten werden (Kaplan A. V. Geschlossene Verletzungen der Knochen und Gelenke. "Medizin", Moskau, 1967, Seiten 156 - 157). Die Bruchstücke bei einem Oberarmbeinhals-Bruch werden mit Hilfe der T-förmigen Platte mit einem hakenförmigen Ende fixiert. Die Operation wird auf folgende Weise durchgeführt: Nach der Gegenüberstellung der Bruchstücke wird der Oberarm ins Innere rotiert. Im Oberarm wird dann eine 5 - 6 cm lange Raste mit einer Zirkularkreissäge eingebracht. Die Raste geht durch die ganze Dicke der Kortikalschicht, die Bruchstelle hinab, nach außen und parallel der Sehne des großen Brustmuskels und dem langen Bizepskopf. Durch diese Raste wird in den Bizepskopf eine schnabelförmige Spitze der T-Platte eingeschlagen. Dann wird die T-Platte in die ausgesägte Raste des Oberarmbeines eingeschlagen. Die Platte wird mit Hilfe von zwei Splinten fixiert, die in die Rinnen der Platte eingeschlagen werden.

Die Nachteile der Osteosynthese mit einer T-förmigen Platte von K. M. Klimov sind:
1) Die Osteosynthese mit der Platte erfordert einen umfangreichen operativen Zugang, bei dem die Abtragung des Deltamuskels vom Schlüsselbein und die umfangreiche Präparierung der Knochenhaut des Oberarmes stattfindet.
2) Der traumatische Zugang und die traumatische Osteosynthese mit der T-Platte bedingen die Gefahr von postoperativen Infektionskomplikationen.
3) In Verbindung mit dem umfassenden Zugang und der traumatischen Osteosynthese durch die T-Platte wird eine hohe Probabilität des Traumas des Nerves Axsilaris vorausgesetzt.

(Kaplan A. V. Geschlossene Verletzungen der Knochen und Gelenke. "Medizin"., Moskau, 1967, Seiten 156 -157).

Ausgehend vom obigen Stand der Technik liegt der Erfindung die Aufgabe zugrunde eine Vorrichtung zur Osteosynthese von Oberarmhalsbein-Brüchen und -Verrenkungen zu schaffen, die die Traumatisierung der Weichteile, sowie die umfangreiche Präparierung der Knochenhaut des Oberarms vermindert, einer Entwicklung von postoperativen Infektionskomplikationen vorbeugt, eine Verletzung des Nerves Axsilaris vermeidet und die Probabilität der Diskolation der Bruchstücke nach der Osteosynthese vermeidet.

Die gestellte Aufgabe wird durch die Merkmale des Anspruches 1 gelöst.

Weitere zweckmäßige und vorteilhafte Ausgestaltungen der Erfindung gehen aus den Unteransprüchen hervor.

Die Resultate werden dank der geringen Abmessungen des Stiftes, der originellen Form, der genügenden Festigkeit und Steifheit der Vorrichtung erreicht. Die Abmessungen des Stiftes ermöglichen die Einführung in die Löcher für die Osteosynthese in den ossär-medullären Kanal. Der Querschnitt des Stiftes bringt eine Festigkeit und eine Steifheit, die für eine stabile Fixierung der Bruchstelle ausreichend sind. Die Länge des Stiftes wird von der Länge des Oberarmkopfes und der Lage der Löcher für die Einführung des Stiftes bestimmt. Der Stift mit einem größeren Querschnitt geht in den ossär-medullären Kanal nicht hinein und ein Stift mit kleinerem Querschnitt hat keine genügende Festigkeit und Steifigkeit. Der vorhandene Knickwinkel (Wölbung) erlaubt den Stift in die Löcher des Knochens einzuführen. Die an den senkrechten Seiten der Schenkel des Stiftes angeformten Erhöhungen bringen eine absolute Fixierung des proximalen und distalen Endes des Stiftes in den Löchern des Knochens, wobei die Spitzen der Schenkel im Oberarmkopf fixiert sind und die Erhöhungen sowohl den mittleren Teil des Stiftes an den Wänden des ossär-medullären Kanal als auch das distale Ende des Stiftes festlegen.

Ein Ausführungsbeispiel der Erfindung ist in den Zeichnungen schematisch dargestellt. Es zeigen:

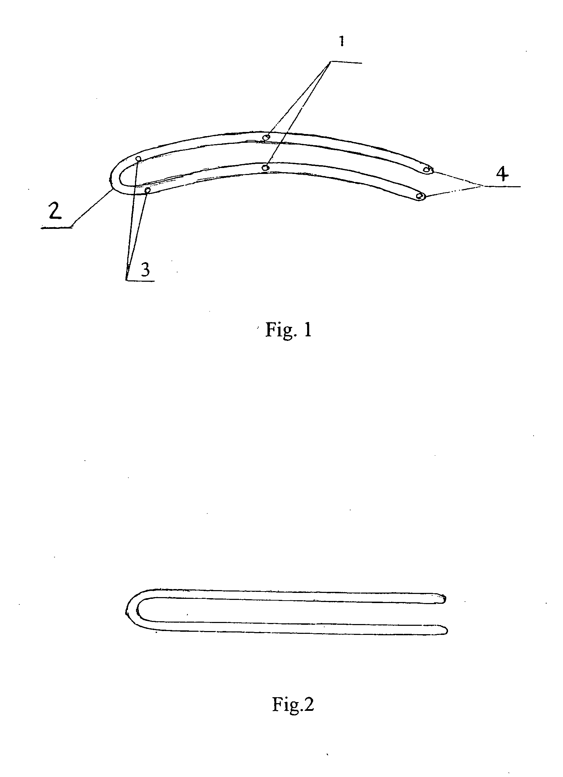

- Fig. 1: eine Ansicht der Vorrichtung in der Frontalebene und
- Fig. 2: die Ansicht der Vorrichtung in der waagerechten Ebene.

In den Zeichnungen zeigen 1 Punkte zur Fixierung der Vorrichtung des mittleren Stiftteils. Mit 2 ist die proximale Stiftspitze gezeigt, die nach der Osteosynthese bis 1 cm über den Knochen herausragt. Die Punkte 3 dienen zur Fixierung des proximalen Stiftteils. Mit 4 sind Punkte am distalen Stiftteil angebracht, so dass auch dieser Stiftteil fixiert werden kann.

Die Vorrichtung wird wie folgt verwendet:
Die Operation wird unter intravenöser Narkose durchgeführt. In der Liegendstellung auf den Rücken besteht Zugang zum oberen Drittel des Oberarmknochens. Die Hand ist dabei an den Körper angelegt und der Ellbogen ist im Gelenk 90° gebogen und auf einem Zusatztisch gelegt. Je nach Oberarmgröße ist das Ausmaß des Zuganges bis 12 cm im Durchschnitt, wobei der Zugang in Richtung der Fasern des Deltamuskels (m. deltoidea) gerichtet ist. Auf dem Wege der Hautdurchschneidung des Unterhautzellstoffes (schichtenweise), der oberflächlichen, eigenen Muskelbinde, der Aponeurose (fascia brachii) des Oberarmes tritt man zum Deltamuskel heraus. Der Muskel wird in Richtung der Fasern des Deltamuskels bis 3 cm durch die weiche, präparierte Klemme auseinander gezogen. Dann wird der Muskel mit zwei Fingern beider Hände der ganzen Länge des Zuganges nach bis zur subdeltaartigen Erstreckung (bursa subdeltoidea) auseinander gedrückt. Man kommt dann zum Bereich des Bruches. Dann wird der Ort für die Einführung des Stiftes vorbereitet. Der Knochen wird von der Knochenhaut präpariert. Dabei tritt man seitwärts der Linie des Oberarmbeinbruches auf der lateralen Oberfläche des Oberarmknochens bis auf den Ansatzpunkt des Deltamuskels. Auf dem Knochen entsteht "das Dreieck" mit der Bruchstelle als Basis. Die Seite rechts davon ist der mediale Rand (margo medialis), die Seite links davon der laterale Rand (margo lateralis). Mit Hilfe des monodonten Retraktors wird die Traktion achsrecht des Oberarmes mit den mit 90° gebogenen Ellbogengelenk durchgeführt. Mit Rotationsbewegungen wird die Reposition des Bruches erreicht. Dem "Dreieckpunkt" näher werden im oberen Drittel des lateralen Vorderteils des Oberarmes (corpus humeri) zwei Löcher querlaufend in einer Entfernung von 1 cm in Richtung des Oberarmkopfes (caput humeri) vom spitzen Winkel zum Knochen bis zum ossär-modullären Kanal angeformt. In die Löcher wird der zum Oberarmknochen kongruent anliegende U-förmige Stift mit dem Rechteckquerschnitt von 0,2 - 0,3 cm eingeführt. Der vorhandene Biegungswinkel des Stiftes ermöglicht es den Stift in die Löcher im Knochen einzuführen. Der Stift rutscht dann dank der Biegung an der Rückwand des ossär-medullären Kanals und springt dann in den Kopf ein, wobei zwei Seiten fixierend wirken. Der Stift wird dann in den Oberarmknochen unter der R-Kontrolle eingeführt. Nach der Osteosynthese ragt die proximale Stiftspitze über den Knochen bis 1 cm heraus. Die Nähte werden auf der Wunde schichtweise angelegt. Es wird ein aseptischer Verband angebracht. Der operierte Teil des Oberarmes wird einen Monat lang mit einem Gipsverband "Dezo" ruhig gestellt. Nach der Beseitigung des Gipsverbandes wird im Laufe von 2 bis 3 Wochen die Ausarbeitung der Kontraktur mit Hilfe der Heilgymnastik bis zur Wiederherstellung der Funktion durchgeführt.

Das Resultat der Behandlung ist zufrieden stellend. Die Funktion der Extremität ist fast vollständig wiederhergestellt.

## Patentansprüche

1. Vorrichtung zur Osteosynthese von Oberarmbeinhals-Brüchen und-Verrenkungen mit einem U-förmigen Stift aus bioniertem Metall, wobei die beabstandeten Schenkel des Stifts am proximalen Ende (2) gewölbt ineinander übergehen und wobei die Schenkel des Stifts der Länge nach gewölbt sind, **dadurch gekennzeichnet,**
**dass** die Schenkel des Stifts einen Rechteckquerschnitt von etwa 0,2 bis 0,3 cm aufweisen und
**dass** an den vertikalen Seiten der Schenkel des Stifts im Abstand vom Bereich des proximalen Endes (2), im mittleren Teil und am distalen Teil des Stifts Fixierelemente (3, 1 und 4) in Form von Erhöhungen angeformt sind.

2. Vorrichtung nach Anspruch 1,
**dadurch gekennzeichnet,**
**dass** die angeformten Erhöhungen zur Fixierung des Stifts einen runden Querschnitt aufweisen und
**dass** der Radius der Erhöhungen etwa der halben Stärke der Schenkel des Stifts entspricht.

3. Vorrichtung nach Anspruch 1,
**dadurch gekennzeichnet,**
**dass** die Schenkel 10 cm lang und mit einem Abstand von 1 cm hergestellt sind und
**dass** der Radius der Wölbung der Schenkel am proximalen Ende (2) des Stifts etwa dem halben Abstand der Schenkel des Stifts entspricht.

## Claims

1. A device for osteosyntheses of fractures and disclocations of the neck of the humerus, having a U-shapted pin ofbionized metal, in which the spaced-apart legs of the pin merge in curved fashion with one another at the proximal end (2) and the legs of the pin are curved longitudinally,
**characterized in that**
the legs of the pin have a rectangular cross section of approximately 0.2 to 0.3 cm, and that on the vertical sides of the legs of the pin, spaced apart from the vicinity of the proximal end (2), fixation elements (3, 1 and 4) in the form of protuberances are integrally formed onto the middle part and the distal part of the pin.

2. The device of claim 1,
**characterized in that**
the formed-on protuberances for the fixation of the pin have a round cross section, and that the radius of the protuberances is equivalent to approximately half the thickness of the legs of the pin.

3. The device of claim 1,
**characterized in that**
the legs are produced with a length of 10 cm and a spacing of 1 cm, and
that the radius of the curvature of the legs on the proximal end (2) of the pin is approximately equivalent to half the spacing of the legs of the pin.

## Revendications

1. Dispositif d'ostéosynthèse suite à des fractures et des luxations de l'humérus, comportant une tige en forme de U en métal biocompatible, dans lequel les branches écartées de la tige passent l'une dans l'autre en formant un arc au niveau de l'extrémité proximale (2), et dans lequel les branches de la tige sont arquées en fonction de leur longueur,
**caractérisé en ce que** les branches de la tige présentent une section rectangulaire d'environ 0,2 à 0,3 cm et
**en ce que** sont façonnés au niveau des côtés verticaux des branches de la tige, à distance de la zone de l'extrémité proximale (2), dans la partie médiane et au niveau de la partie distale de la tige, des éléments de fixation (3, 1 et 4) sous la forme de bosses.

2. Dispositif selon la revendication 1, **caractérisé en ce que** les bosses formées présentent, pour la fixation de la tige, une section ronde, et
**en ce que** le rayon des bosses correspond à environ la moitié de l'épaisseur des branches de la tige.

3. Dispositif selon la revendication 1,
**caractérisé en ce que** les branches sont fabriquées avec une longueur de 10 cm et un écartement de 1 cm, et
**en ce que** le rayon de courbure des branches au niveau de l'extrémité proximale (2) de la tige correspond à environ la moitié de l'écartement des branches de la tige.
